Europäisches Patentamt

European Patent Office

Office européen des brevets

(11) Publication number: **0 155 224**
A2

(12) **EUROPEAN PATENT APPLICATION**

(21) Application number: **85630029.8**

(22) Date of filing: **14.03.85**

(51) Int. Cl.⁴: **G 01 N 33/543**, G 01 N 33/545, G 01 N 33/76, G 01 N 33/74

(30) Priority: **15.03.84 US 589723**

(43) Date of publication of application: **18.09.85**
**Bulletin 85/38**

(84) Designated Contracting States: **AT BE CH DE FR GB IT LI LU NL SE**

(71) Applicant: **CHROMAGENICS, Inc., 1 Walnut Street, Boston Massachusetts 02108 (US)**

(72) Inventor: **Crockford, David R., 220 Kenooza Avenue, Haverhill Massachusetts 01830 (US)**

(74) Representative: **Schmitz, Jean-Marie et al, Office Dennemeyer S.à.r.l. 21-25 Allée Scheffer P.O. Box 41, L-2010 Luxembourg (LU)**

(54) Solid-borne complex bearing chromagen responsive functionality for antibody, antigen, receptor, or ligand detection.

(57) Disclosed is a reagent useful in the determination of a component of an antibody-antigen or a receptor-receptor binding molecule reaction. The reagent is characterized by the reaction product selected from the group consisting of:

(a) a solid support matrix having the antibody linked thereto and a conjugate of the antigen and a chromagenically responsive marker;

(b) a solid support matrix having the antigen linked thereto and a conjugate of the antibody and a chromagenically responsive marker;

(c) a solid support matrix having the receptor linked thereto and a conjugate of the receptor binding molecule and a chromagenically responsive marker; and

(d) a solid support matrix having the receptor binding molecule linked thereto and a conjugate of the receptor and a chromagenically responsive marker. The reagent is especially useful in the detection of hCG in order to ascertain whether a female is pregnant. For such embodiment, reagent (a) is composed of the reaction product of nylon having anti-β-hCG linked thereto which is bound to a conjugate of hCG and an enzyme, e.g. horseradish peroxidase, by a carrier molecule such as, for example, Ficoll₇₀ or bovine serum albumin. Various other non-isotopic immunoassay tests are discussed and disclosed for the reagent.

ACTORUM AG

## SOLID-BORNE COMPLEX BEARING
## CHROMAGEN RESPONSIVE FUNCTIONALITY
## FOR ANTIBODY, ANTIGEN, RECEPTOR, OR LIGAND DETECTION

### Background of the Invention

The present invention is related to antibody, antigen, receptor, and ligand assay techniques and more particularly to an improved solid-borne conjugate complex containing a chromagen responsive marker for determination and detection of an antibody, an antigen, or a receptor.

An important tool in clinical diagnostics has been radioimmunoassay and radio receptor assay because of the sensitivity and specificity which they provide to the diagnostician. Radioassay procedures, however, require not only the materials necessary to implement the radioisotope labelling but also require sophisticated machinery for radioactive isotope detection. Such procedures, then, are limited to properly equiped laboratories in communities and countries which can provide the necessary sophistication required for proper implementation of such assay techniques. Consequently, a number of medical situses are precluded from performing assay tests having the sensitivity which radioassay procedures provide. Such situses include physician offices, urgent care clinics, rurally-located hospitals and medical centers, and like environments. Moreover, rapid and simple home detection procedures are not viable either.

The development of non-isotopic immunoassay, such as enzyme immunoassay, has been an approach adopted to permit determination and detection of drugs, steroids, peptides, etc. without resort to handling radioisotopes and having access to radioactive isotope detection equipment therefor.

An example of implementation of such enzyme immunoassay technology is the advent of in-home pregnancy testing where a woman can determine whether she is pregnant by employing a relatively simple assay technique. Prior efforts at developing pregnancy testing methods can be found in U.S. Pats. Nos. 4,376,110, 4,088,749, 4,016,250, 4,094,963, 3,862,302, 3,655,838 and 3,654,090, Van Weeman et al. "Immunoassay Using Antigen-Enzyme Conjugate", FEBS Letters, 15, 232 (1971), Saxena et al., "Isolation of the Luteinizing Hormone-Chorionic Gonadotropin Receptor in High Yield from Bovine Corpora Lutea", The Journal of Biological Chemistry, Vol. 258, No. 5, pp 3410-3158 (March 10, 1983); Saxena et al.,

"Radioreceptorassay of Luteinizing Hormone-Human Chorionic Gonadotropin in Urine: Detection of the Luteinizing Hormone Surge & Pregnancy", Fertility & Sterility, Vol. 28, No. 2 (Feb. 1977); and Saxena et al. "Diagnosis and Management of Pregnancy by the Radioreceptorassay of Human Chorionic Gonadotropin", American Journal of Obstetrics and Gynecology, Vol. 131, pp 97-109 (May 1, 1978). Specific diagnostic test devices for implementation of such tests can be found in U.S. Pat. No. 3,579,306 and U.S. Design Pat. No. 215744, for example. The test methods disclosed in such art generally employ hCG binding reactions with antibodies (immunoassay), receptors (receptorassay), and like techniques. It should be recognized that the techniques developed for pregnancy testing (radioassay and enzyme assay) similarly apply in the assay of other molecules, including, but not limited to, steroidal molecules, protein molecules, hormones and the like. The art recognizes the broad applicability of such enzyme immunoassay techniques as those skilled in this art will appreciate. Notwithstanding the developments currently occurring in this field, prior tests still lack the degree of sensitivity for detecting extremely low levels of antibody, antigen, or receptor which is necessary in order to more equilibrate the enzyme immunoassay techniques with radioimmunoassay techniques. Also, these prior tests which approach radioassay sensitivity require the use of equipment and instrumentation. A more sensitive chromagenically responsive complex for such assay techniques is the subject matter of the present invention.

## Broad Statement of the Invention

One aspect of the present invention is a reagent for the determination and detection of one component of an antibody-antigen reaction. Two basic forms of such reagent are encompassed within the precepts of the present invention. One form is an insoluble solid support matrix or substrate having an antibody chemically linked thereto which is in association with a conjugate of the antigen and a carrier bearing a multiplicity of chromagenically responsive marker molecules. The second form of the reagent is an insoluble solid support matrix or substrate having the antigen chemically linked thereto which is in association with the conjugate of an antibody and a carrier bearing a multiplicity of chromagenically responsive marker molecules. A preferred chromagenically responsive marker is an enzyme. For pregnancy determination, the antigen is hCG and the antibody is anti-$\beta$-hCG $\gamma$-globulin.

Another aspect of the present invention is a method for the determination of one component of an antibody-antigen or a receptor-receptor binding molecule (ligand) reaction. Such method is an immunochemical assay method employing the novel reagent disclosed above. Such method comprises contacting a sample containing the component to be determined with a determined amount of the foregoing reagent for a time sufficient to permit the immunochemical reaction to occur, separating the insoluble substrate of the reagent from the test solution, and treating said remaining test solution with a chromagen responsive with said marker. In pregnancy testing, for example, the presence of the hCG hormone in the body fluid sample tested will be indicated by an appropriate color appearance or change depending upon the particular chromagen system utilized. Further aspects of the present invention include a reagent and method for determination of a receptor or its receptor binding molecule. Antigens and antibodies, and receptors and receptor binding molecules are sterically and biologically specific reactants which can be determined in accordance with the present invention, though the present invention ultimately may find utility for assay of other biologically specific reactants.

Advantages of the present invention include the development of a reagent which has many more markers affixed to each molecule of reagent than was heretofore possible. Increased marker content permits a greater sensitivity of the test in determination of lower levels of the substance to be detected and/or measured. In pregnancy testing, for example, another advantage is the ability to detect pregnancy at a much earlier stage than was heretofore possible using enzyme immunoassay. A further advantage is the greater reliability of the test method and reagent of the present invention in the determination of an antigen, antibody, or receptor. A yet further advantage is the development of a reagent which is stable and is preformed which permits excellent long-term storage and handling of such reagent. A still further advantage is the simplicity in implementing assays with the novel reagent which will permit greater utilization of it by non-skilled users, eg. individuals at home. These and a variety of additional advantages will become readily apparent to those skilled in this art based upon the disclosure contained herein.

Brief Description of the Drawings

Fig. 1 is a diagrammatic representation of the reagent of the present invention which additionally shows the displacement reaction for determination of, for example, hCG in urine from a pregnant woman; and

Fig. 2 is a diagrammatic representation of the displacement product resulting from the determination reaction of Fig. 1.

The drawings will be described in greater detail in connection with the Detailed Description of the Invention which follows.

## Detailed Description of the Invention

The present invention provides a reagent and a method for conducting diagnostic tests utilizing visible indicia rather than conventional laboratory equipment, eg. spectrometer, spectrophotometer, etc. The diagnostic tests which have been developed in accordance with the invention permit home use by lay (unskilled) people. Moreover, such diagnostic tests are ideal for use in non-laboratory medical settings such as a physicians office, medical outpatient clinics, or emergency rooms. Reliable assays which are extremely accurate and sensitive now may be rapidly and relatively inexpensively conducted in these settings. Since a visible indicia, eg. color change, color formation, or pattern formation, is utilized, a trained laboratory technician no longer need perform and evaluate the results of the diagnostic test. Notwithstanding such qualitative characteristics of the diagnostic test, quantification of results can be readily ascertained as will be explained more fully below. The novel reagent of the present invention which permits the determination of one component of an antibody-antigen reaction or a receptor-receptor binding molecule reaction is formed from five basic components. These components include a solid support matrix, an antibody (or receptor), an antigen (or receptor binding molecule), a carrier molecule, and a chromagenically-responsive marker or tracer molecule. Of special significance is the formation of a single component or complex reagent, a ready distinction over the art. While a wide variety of reaction techniques may be envisioned for formation of the reagent, several distinctly preferred reaction specific procedures will be described below. In particular, the procedures will specifically refer to use of human chorionic gonadotropin (hereinafter hCG) and its use in pregnancy diagnosis. It should be appreciated, however, that such specific description is merely by way of illustration and not by way of limitation of the invention.

Referring initially to the solid phase or support matrix, preferably the matrix is comprised of a solid which can be treated for the covalent linking of either the antibody, receptor or the antigen thereto. A preferred matrix is nylon-6; however, other suitable conventional solid phases include, for example, other nylons, polybutadiene, polystyrene, latices, butadiene-styrene copolymers, glass, agarose, crosslinked dextran, cellulose, liposome (phospholipid bilayer membrane), filter paper, and the like. The support matrix may be manufactured in various forms, including, for example, rods, plates, rings, discs, strips, tubes, beads, cones, particles, etc. Maximization of the surface area of the support is desirable for providing a maximum concentration of the reagent. While physical adsorpotion techniques for linking the antibody or other reagent to the solid matrix may prove acceptable for some uses, most preferably, the solid matrix will be treated for the covalent linking of either the antibody, receptor, or the antigen thereto. For example, a nylon-6 solid may be aminated to attach amine groups eg. with HCl or 1,6-diamino hexane, followed by thiolation, eg. with N-acetyl homocysteine thiolactone (AHTL), for converting such added amine groups into thiol groups. The use of AHTL as a spacer molecule between the solid matrix and an antibody, for example, reduces steric hindrance of the thiol reactive groups and the antibody binding sites. The thiolated carrier then can be linked with, for example, a maleimidated antibody, eg. anti-hCG. At a pH of less than 7, amine groups are unreactive with thiol groups so that cross-linking is prevented by this procedure. Additional methods for accomplishing the covalent linking of an antibody or antigen to a solid phase can be found in Kawoi et al., "An Improved Method of Conjugation of Peroxidase with Protein", Fed Proc., 32 Abstract 840 (1973); Saxena et al., "Development of a Solid-Phase Centrifugation-free Enzyme Assay for LH Ovulation Detection", Psycho-neuroendocrinology in Reproduction, Zichella et al., eds., Elsevier/North Holland (1979); and Post et al. "A Rapid, Centrifugation-free Radioimmunoassay Specific for Human Chorionic Gonadotropin Using Glass Beads as Solid Phase", J. Clin. Endocrinol. Metab. 50, 169 (1980). Accordingly, appropriate use of aldehydes, carboxyl groups, and other chemically reactive groups or covalently linking the antibody, receptor, or antigen to the solid phase can be used as is necessary, desirable, or convenient, in conventional fashion.

As noted above, the second component of the reagent is the antibody, receptor, or antigen which has been treated (modified) for its reaction onto the solid phase. For present purposes, antibody is to be construed broadly to include a single monoclonal antibody; a single polyclonal antibody; a mixture of two or more monoclonal antibodies (in a predetermined ratio), each of which has different binding and cross-reacting characteristics to the same antigen; a mixture of two or more polyclonal antibodies (in a predetermined ratio), each of which has different binding and/or cross-reacting characteristics to the same antibody; or a mixture of one (or more) monoclonal antibodies and one (or more) polyclonal antibodies, each of which has different binding and/or cross-reacting characteristics to the same antigen. Such antibody mixtures may display a cooperative effect towards the same antigen (eg. hCG), but presumably to different antigenic determinants (epitopes) of that antigen. Of course, a full appreciation of this cooperative effect was absent prior to the advent of monoclonal antibodies. Receptors suitable for use in the immunodiagnostic test of the present invention are to be construed broadly in accordance with the foregoing discussion. See also Moyle et al., "Use of Monoclonal Antibodies to Subunits of Human Chorionic Gonadotropin to Examine the Orientation of the Hormone and Its Complex with Receptor", Proc. Natl. Acad. Sci. USA, Vol. 79, pp 2245-2249 (April 1982); Ehrlich et al., "Mixing Two Monoclonal Antibodies Yields Enhanced Affinity for Antigen", The Journal of Immunology, Vol. 128, No. 6 (June 1982); and Ehrlich et al., "Cooperative Immunoassays: Ultrasensitive Assays with Mixed Monoclonal Antibodies", Science, Vol. 221, pp 279-281 (July 15, 1983). In this regard, the term "antigen" must be construed broadly to include not only an antigen of an antigen-antibody pair, but also an antibody of an antibody-antibody pair where a second antibody to a first antibody is developed. The first antibody (eg. an anti-$\gamma$-globulin) is equivalent to an antigen for present purposes. Such antibody-antibody pairs have been used, for example, where an antigen is scarce, eg. in short supply and/or expensive. Preparation of a reagent in accordance with the precepts of the present invention based on such antibody-antibody pairs certainly is within the scope of the present invention.

For pregnancy testing, it is presently preferred to link the antibody to the nylon or other solid matrix, though the linking of the antigen or receptor to the solid phase is appropriate. Regardless of which component of the antibody-antigen reaction, or the receptor-receptor binding molecule reaction, is linked to the solid phase, such component will be associated with a labelled conjugate for formation of the reagent of the present invention. The antibody-antigen binding (or coupling) is not a covalent bond, but is more in the nature of Van der Waals bond which can be displaced by the component to be determined.

Accordingly, the remaining three ingredients preferably are conjugated and the conjugate added to the treated solid matrix. Conjugation is a procedure for predictably coupling a proteinaceous material or a non-proteinaceous material with another proteinaceous material or a non-proteinaceous material. For the reagent of the present invention, an ideal conjugate bears one or two molecules of antigen, receptor, or antibody (depending upon the component linked to the solid phase) and a multiplicity of chromagenically-responsive marker or tracer molecules. For example, conventional reagents which directly covalently link an antibody or antigen to an enzyme can only provide a maximum of three or four molecules of enzyme per molecule of antigen or antibody. Clearly, the amount of enzyme present will determine the sensitivity of the assay in the ability to detect the presence of the desired component in lower proportions. This translates in the ability of the reagent of the present invention to permit the detection (visible indicia) of pregnancy no later than the expected commencement of the female's period. The use of the conjugate in accordance with the precepts of the present invention can permit broadly from about 1 to 15, for example, molecules of enzyme per molecule of hCG antigen in the conjugate and typically between about 5 and 10 molecules of enzyme per molecule of antigen is borne by the conjugate. It should be recognized that various enzymes useful as tracers or markers include horseradish peroxidase, alkaline phosphatase, catalase, and the like. Enzymes are the marker of choice since they can be readily detected in very small amounts. It should be understood, however, that additional markers including, for example, phenols, enzyme cofactors, dyes (eg. azo dyes), chemiluminescence precursors, enzyme substrates, fluorochromes, fluorogens, fluorescent quenchers, and hemes, may be used as the marker in formation of the conjugate. See, for example, Schall and Tenoso, "Alternatives to Radioimmunoassay: Labels and Methods", Clin. Chem, Vol. 27, No. 7 (1981) An appropriate marker for use in the conjugate need only be reactive with a chromagen for providing a visible indicia of its presence.

The carrier molecule of the conjugate must be soluble in the reaction medium, typically an aqueous medium, which places a practical limit on the molecular weight of such carrier. Further, carriers too high in molecular weight exhibit protracted reaction kinetics which makes their use impractical. Preferred carriers tend to have a molecular weight not substantially in excess of about 70,000 and such carriers include, for example, sucrose polymers (eg. $Ficoll_{70}$), bovine serum albumin (hereinafter BSA) and like known carrier molecules. A variety of conjugation techniques which are reaction specific have been proposed in the art and can be found in the following references: Engvall et al., "Enzyme-linked Immunoabsorbent Assay (ELISA), Quantitative Assay of Immunoglobulin G", Immunochemistry, 8, 871 (1971); Kawoi et al., "An Improved Method of Conjugation of Peroxidase with Protein", supra; Saxena et al. "Development of a Solid-Phase Centrifugation-free Enzyme Assay for LH for Ovulation Detection", supra; Carlsson et al., "Protein-Thiolation and Reversible Protein-Protein Conjugation", Biochem J., 173, 723 (1978); Khan et al. "Use of Purified Gonadotropin Receptor in the Development of an Enzyme Receptor Assay (EA) for Luteinizing Hormone and Human Chorionic Gonadotropin", Enzyme Labelled Immunoassay of Hormones and Drugs, S.B. Pal, ed.

de Gruyler & Co (1978); and Lee et al., "A Method for Preparing Beta-hCG COOH Peptide-Carrier Conjugates of Predictable Composition", Molecular Immunology, 17, 749-756 (1980).

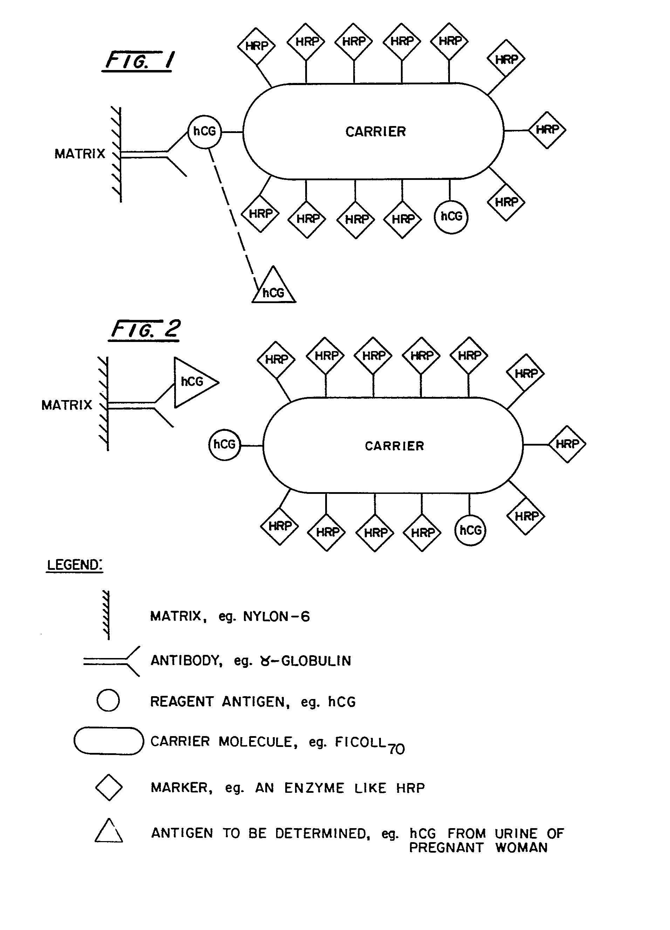

With specific referece to the drawings, a schematic representation of the reagent of the present invention with specific reference to the determination of hCG from the urine of a pregnant woman is set forth. It must be remembered that the drawings are merely illustrative and are intended to provide an enhanced visual representation of the reagent of the present invention. Referring to Fig. 1, the solid matrix is seen to bear a rather complex molecular structure consisting of an antibody connected to hCG which is connected to a carrier molecule. The carrier molecule bears an unassociated hCG molecule as well as several molecules of marker which in this case is horseradish peroxidase. The visual representation of the reagent illustrates several important aspects of the present invention. Initially, it will be seen that the carrier molecule typically bears one or two molecules of antigen, eg. hCG, per molecule of carrier. Having more than one molecule of hCG per molecule of carrier enhances the reaction rate and reactivity of the carrier molecule for linking with the antibody affixed to the matrix. Next, it will be seen that the ratio of HRP marker molecules per molecule of hCG is represented to be 6:1, which is within the broad range of 4-15:1 referred to above. Of importance is that the release of one molecule of conjugate, in accordance with the specific structure set forth in the drawings, provides 12 molecules of HRP for each molecule of hCG displaced. The sensitivity of the reagent is enhanced by this high ratio of marker per molecule of antigen to be determined. It will be appreciated, of course, that the matrix bears a myriad of such molecules set forth in Fig. 1 as the particular drawing is illustrative by showing only one. Also of importance is that the antibody matrix bond is a covalent bond which is recalcitrant to severing under typical reaction conditions to which the reagent is to be exposed. The hCG to be determined, eg. from the urine of a pregnant woman, is the triangular hCG symbol with the dotted line representing the displacement reaction which occurs when the matrix is exposed to the urine of a pregnant woman. The hCG molecules to be determined are in the liquid urine whereas the reagent is a solid form. In Fig. 2, the resulting displacement reaction has occurred. The solid matrix with covalently linked antibody now has the hCG molecule to be determined from the pregnant urine associated with the antibody. It is this solid matrix form which is removed from the remaining liquid. The carrier molecule containing the HRP marker molecules now is in the liquid which remains after the matrix is removed. It is this carrier molecule displaced by the hCG molecule to be determined that is assayed by visual perception upon the addition of appropriate chemicals to detect the presence of the HRP molecules. It will be seen that the resulting carrier molecule has a great number of HRP marker molecules for detection per molecule of hCG displaced from the matrix. The remarkable sensitivity of the invention is, thus, obtained.

Two representative general reaction schemes for conjugate formation will be briefly outlined here and their details will be provided in the examples which follow. Regardless of which scheme is implemented, the antibody, antigen, or receptor and the chromagenically responsive marker are modified to react or combine with the carrier molecule. In their modified state, there is no association or coupling between the marker and the antibody, antigen, or receptor. Again, such reaction procedures are illustrative and preferred, but are not a limitation of the invention. The first reaction scheme can be referred to as the formation of a "sandwich" wherein the enzyme or other marker is built up successively in layers to increase the quantity of marker per molecule of carrier. It should be noted that the ability to increase the number of moles of enzyme or marker on most carriers is limited more by steric hindrance than by number of reactive sites. The layering or sandwiching technique is one method for overcoming such limitation. Basically, two schemes can be envisioned for readily implementing this reaction scheme. One scheme involves the maleimidation of the carrier molecule and the thiolation of the marker and antigen followed by the reaction of these components simultaneously or sequentially in the desired proportions. This scheme tends to result in difficulty in purification without cross-linking.

Alternatively, the carrier may be thiolated, and the antigen and marker maleimidated followed by their subsequent reaction. This scheme results in easier purification without cross-linking. The preferred scheme can be implemented, for example, as follows. The BSA or other carrier is thiolated utilizing N-acetyl homocysteine thiolactone (hereinafter AHTL). Horseradish peroxidase (hereinafter HRP) or other enzyme is maleimidated with 6-maleimido caproic acid N-hydroxyl succinimide ester (hereinafter MCS). The thiolated BSA may be reacted with up to 4 to 5 moles of maleimidated HRP. This reaction product then may be reacted with a dithiol such as 1,6-dithiohexane or dithiothritol, for example, followed by the addition of more maleimidated HRP. This latter sequence may be repeated as many times as is necessary for providing additional moles of enzyme marker per mole of BSA carrier. Subsequently in this reaction scheme, the maleimidated hCG (using MCS) then is reacted with the HRP sandwiched carrier molecule in a proportion of about 1 to 2 moles of maleimidated hCG per mole of thiolated BSA. The resulting conjugate now may be associated with the antibody treated nylon or other solid phase for forming the novel reagent of the present invention.

A variation on the "sandwich" scheme described above, which utilizes controlled reactions, involves the formation of an enzyme (or other marker) polymerizate. Such polymerization is a non-specific reaction involving cross-linking, which cross-linking reaction sequence has been avoided in other techniques described herein. Enzyme cross-linking can utilize carbodimides, glutaraldehydes, multi-isocyanates, and like bivalent and polyvalent agents for building an enzyme-rich polymerizate. Such polymerizate, then, can be utilized in the conjugate formation by various of the specific reaction sequences set forth herein for formation of an antigen, antibody, or receptor conjugate rich in enzyme or other marker.

The second broad reacton scheme noted above can be implemented by two different methods. This scheme utilizes N-succinimidyl-3-(2-pyridylthio)-propionate (hereinafter SPDP) reported in Carlsson et al., "Protein-Thiolation and Reversible Protein-Protein Conjugation" (supra). This reaction scheme can be implemented utilizing, for example, $Ficoll_{70}$ as the carrier molecule which is reacted with between about 5 and 20 molecules of SPDP per molecule of $Ficoll_{70}$. The hCG or other antigen also is reacted with SPDP to provide one or two thiol groups per mole of hCG. The SPDP-modified $Ficoll_{70}$ and SPDP-modified hCG then may be reacted in a proportion so that the Ficoll molecule only contains one or two hCG molecules per molecule of carrier. Next, the HRP or other marker may be thiolated utilizing SPDP or AHTL. The thiolated HRP then is reacted with the $hCG-Ficoll_{70}$ adduct to provide a conjugate containing one or two molecules of hCG per molecule of Ficoll and between about 4 and 15 molecules of HRP per molecule of Ficoll. The second method of implementing this second reaction scheme involves the separate thiolation with SPDP of the hCG, HRP and $Ficoll_{70}$ followed by the simultaneous reaction of these three reactants. Again, their molar proportion is the same as that utilized in the initial reaction method described herein. Regardless of the method employed, the resulting conjugate then is associated with the antibody treated nylon or other solid matrix to form the reagent of the present invention. Such reagent is stable and can be preformed for ease of handling and long-term storage stability. Also, the reagent does not link with free amines.

The conjugate is associated with the antibody treated plate by permitting these reactants to come into contact for a time period typically ranging from about 2 hours to 5 days and more often about 12 hours, depending upon temperature. The resulting complex affixed to the solid phase then is briefly washed to remove surface tension-bound conjugates. The solid phase, however, is not soaked in water in order to prevent loss of the conjugate. It should be noted that aqueous prepared complexes tend to be more easily solubilized in water, thus the need for minimizing the contact period between the solid bearing complex and water. Finally, in order to suppess dehydration of the complex on the surface of the solid phase, a protective coating, propylene glycol-ethylene glycol, mannitol, or the like may be applied to the solid phase by dipping or other conventional technique. The resulting solid phase bearing reagent then is air dried and is ready for use.

While specific reagents have been referred to in the description above, it should be recognized that such description is illustrative only as various of the reagents herein may be interchanged and use of other reagents is permissible also. Also, while much of the description of the reaction procedures has focused on determination of pregnancy, it should be noted that other specific uses of the reagent can be contemplated. For example, if palpation of the uterus or ultrasound sonography shows no presence of an implanted fetus, quantification of hCG is important in order to ascertain whether an ectopic pregnancy exists. Quantification is practiced using pre-determined dilution data of known concentrations. Very low hCG production, consistent with an ectopic pregnancy, can be readily determined by the reagent of the present invention due to its extreme sensitivity by having a large proportion of enzyme or other marker per molecule of reagent. Other uses of the reagent involve the determination of steroidal molecules, protein molecules, and hormones, for example. In this regard, the reagent should find utility in ovulation and infertility detection by its application to estrogen, progesterone, and luteinizing hormone production in the female which can be determined from body fluids including urine and blood. Again, the sensitivity of the reagent of the present invention even permits the use of saliva, for example, in which progesterone and estrogen detection has been reported. Another use involves the use of the reagent for pregnant women which have a history of aborting. The proportion of estriol may be readily monitored by the physician or the woman herself in order to determine whether a drop in estriol level occurs. A drop in the estriol level is indicative of pregnancy problems, eg. a placenta not functioning properly.

In order to amplify on other specific uses of the inventive reagent, reference is made to ovulation testing which traditionally has utilized LH detection (as noted above). The sensitivity and flexibility of the inventive reagent permits determination of both estrogen and LH for ovulation prediction and detection (pinpointing), respectively. The inventive reagent could use, for example, alkaline phosphatase as the enzyme marker for estrogen and HRP as the enzyme marker for LH. Of course, a mixture estrogen and LH, and their corresponding antibodies, would be used in synthesis of the inventive reagent. The assay would employ sequentially, for example, indoxyl phosphate as the chromagen for estrogen (blue color development indicates a positive result) and then aminoantipyrine as the chromagen for LH (red color development indicates a positive result). Development of a resultant purple color would indicate the presence of both estrogen and LH. Advantages of such a dual detection scheme include the development of unique colors (and/or patterns) as well as the ability with one or more chromagenically-responsive markers to use chromagens, which alone, develop weak or difficult-to-detect color. Also, incidence of false results should be diminished because of the need to determine two (or more) hormones (other molecules). Thus, the use of mixtures and combinations of the various ingredients of the inventive reagent is within the precepts taught herein.

The following examples show how the present invention has been practiced, but should not be construed as limiting the present invention. All units in this application are in the metric system and all references cited herein are expressly incorporated herein by reference.

## EXAMPLES

### EXAMPLE 1

#### Preparation of Anti-hCG Labeled Nylon-6 Matrix

Nylon-6 plates (0.317 by 1.27 by 10.16 cm) were soaked in a 3 N HCl solution for 1 hour at 37°C. The plates then were washed extensively with running tap water, and then rinsed three times with distilled water. The aminated plates were thiolated by 0.05 molar AHTL in a 1 molar solution of imidazole (nitrogen saturated) overnight. The thiolated plates were washed repeatedly with 0.2 molar acetic acid until no thiol compound in the washing liquid was detected by Elmens reaction.

Anti-$\beta$-hCG (prepared and purified by conventional methods) was lightly maleimidated (around 1 to 2 maleimide groups per mole of $\gamma$-globulin) with MCS, purified by gel filtration, and lyophilized.

The thiolated nylon-6 plates were soaked in nitrogen-saturated 0.1 molar succinate buffer, pH 6.0, containing 0.04% EDTA, and maleimido-$\gamma$-globulin (0.2

milligrams/milliliter). The soaking plates were agitated in the solution at room temperature overnight. The plates then were immersed in a 15% sodium chloride solution containing N-ethyl maleimide to block any free-SH groups remaining on the surface of the plates. After one-half an hour immersion, the plates were removed and washed once with a 15% sodium chloride solution, and then with water for a sufficient number of times required to reach the point at which the washing liquor no long absorbed at 280nm.

The antigen binding capacity of the plates was determined by radioimmunoassay using $^{125}$IhCG having a known specific activity.

## EXAMPLE 2

### HRP-BSA-hCG

#### Monomeric HRP Preparation

Amino groups on the surface of the hCG molecule were extensively maleimidated with MCS followed by purification by gel permeation chromatography. The HRP enzyme was briefly thiolated with AHTL to contain about 1 to 2 thiol groups per mole of HRP (using Fluoroscamin-Fluorometric method to follow the progress of the thiolation reaction). The thiolated enzyme product was dialyzed against 0.2 molar acetic acid and lyophilized. The thiolated hCG product and the thiolated HRP enzyme both were dissolved in nitrogen-saturated 0.1 molar succinate buffer, pH 6.0, containing 0.04% EDTA. The resulting conjugate was purified by Sephacryl S-200 column.

#### HRP-BSA Copolymer Preparation

Approximately one-fifth of the amino groups of BSA were thiolated with AHTL. The thiolated-BSA was dialyzed against 0.2 M acetic acid and lyophilized. Next, HRP was maleimidated with MCS to a degree such that each HRP enzyme molecule contained about 3 maleimido groups. The modified proteins then were reacted in $N_2$-saturated 0.1 M succinate buffer, pH 6.0, 0.4% EDTA, to produce a BSA molecule containing about 4-5 molecules of HRP per BSA molecule. The increased weight of the polymeric product permitted its separation from the monomeric reactants by conventional gel permeation chromotography.

The separated BSA-HRP copolymer was reacted with 1,6-dithiohexane (or alternatively dithiotritol) to introduce one or two thiol groups in each of the HRP molecules attached to a BSA molecule. This thiolated product was reacted with additional maleimidated HRP (as prepared above). The resulting product was separated from monomeric HRP by gel filtration and lyophilized. This procedure can be repeated as many times as necessary to produce a multi-layered HRP-BSA product.

The hCG molecule was maleimidated briefly with MCS to contain one to two equivalents of maleimide groups per mole of hCG. The multi-layered HRP-BSA product then was reacted with dithiothreitol (or alternatively 1,6-dithiohexane) to introduce -SH groups on the last layer of HRP molecules. The maleimidated hCG and the thiolated HRP-BSA product (1:10 molar ratio, respectively) then were dissolved in $N_2$-saturated 0.1 M succinate, pH 6.0, 0.04% EDTA, and the product separated by gel filtration. The HRP to hCG ratio in the enzyme-labelled hormonal conjugate product was determined by spectrophotometry for HRP (substrate was 4-aminoantipyrine in phenol solution mixed with dilute hydrogen peroxide in phosphate buffer, pH 7.0) and RIA for hCG.

Several of the $\gamma$-globulin labelled nylon plates were soaked separately in each of the hCG-BSA-HRP conjugates prepared above containing 0.2% BSA-PBS solution (about 1-7 IU value of conjugate per milliliter of BSA solution) for 5 days to saturate the antigen binding sites.

## EXAMPLE 3

### HRP-Ficoll-hCG

#### Method A:

Aminohexyl-Ficoll$_{70}$ dissolved in a minimum volume of 0.1 M borate buffer, pH 8.5, was reacted with a 20 molar excess of N-succinimidyl-3-(2-pyridylthio)-propionate (SPDP dissolved in DMF) at room temperature for 45 min. The thiolated Ficoll$_{70}$ product was purified by gel filtration through a Sephadex G-25-water column and the material collected from the void volume of the column was lyophilized. The degree of SPDP modification was determined spectrophotometrically using a molar extinction coefficient of $8.08 \times 10^3 \ M^{-1} \ cm^{-1}$ for 2-thiopyridone which is released from Ficoll$_{70}$ upon reaction with a thiol compound, eg. cysteine. The optimum number of SPDP groups ranges from between 5 and 20 equivalents per mole of Ficoll$_{70}$.

The thiol derivative of hCG was prepared in the manner described previously for the thiolation of HRP (eg. utilizing SPDP as the thiolating reagent). The degree of thiolation was monitored fluorometrically by following the amine group consumption in the carrier molecule. The optimum degree of thiolation is 1 eq. of -SH per eq. of hCG. The thiolated hCG product was gel filtered through a Sephadex G-25 column in 0.2 M acetic acid solution. The purified thiolated hCG collected from the void volume of the column was analyzed for total mass recovered and total -SH content by spectrophotometry. The product was adjusted to pH 4.7 by the addition of sodium bicarbonate under a nitrogen blanket for use in coupling with the thiolated Ficoll prepared above.

Sufficient lyophilized SPDP-Ficoll was added to the pH adjusted thiolated hCG solution to yield a molar ratio of 1:2, respectively. This reaction was conducted under a $N_2$ blanket at room temperature and the progress of the reaction monitored spectrophotometrically (O.D. 343 n.m.) by following the release of 2-thiopyridone. When the O.D. 343 n.m. reached a maximum, the reaction mixture was concentrated to approximately 25 mg/ml by ultrafiltration through a PM10 membrane.

HRP dissolved in 1 M sodium bicarbonate solution was citracconylated at its amino groups, oxidized by the addition of sodium periodate to remove a portion of its carbohydrate content and to create aldehyde groups, which groups then were subjected to reductive amination with a 2 M solution of 1,6-diaminohexane. The product then was desalted and lyophilized. The aminated HRP was thiolated by AHTL to a degree such that each mole of $HRP-NH_2$ received only one mole of AHTL. The thiolated HRP was purified by gel filtration through a 0.01 M AcOH-Sephadex G-25 column. The void volume material collected was pH adjusted to 4.7 by the addition of sodium bicarbonate powder under a $N_2$ blanket.

Finally, the thiolated HRP solution was mixed with the concentrated hCG-Ficoll conjugate solution (pH 4.7) and the mixture shaken under a $N_2$ blanket at room temperature for 16 hr. (molar ratio of hCG:Ficoll:HRP was 2:1:(4-15)). The reaction was terminated with the addition of sufficient cysteine to convert residual free SPDP groups on the $Ficoll_{70}$ molecule to unreactive S-cysteinyl-S-propionyl adducts. The product was purified by gel permeation chromatography through a Sephacryl S200 column in 0.2 M ammonium sulfate, 0.05 M sodium phosphate, pH 6.8, with removal of uncoupled hCG, HRP and cysteine. The void volume material collected from the column was analyzed for hCG content by RIA and for HRP content by spectrophotometry. The preparation was diluted to around 1.0 IU per ml with 0.05% TWEEN 20-0.1 M sodium phosphate, pH 6.8.

Method B

The amino-HRP and hCG molecules each were separately thiolated using SPDP as described above, the thus-prepared thiolated derivatives purified and lyophilized. The amino-hexyl-Ficoll$_{70}$ reagent was thiolated with AHTL (optimum degree of thiolation being 10 to 20 equivalents per mole of amino hexyl-Ficoll$_{70}$), and the product purified by gel filtration through a 0.1 molar acetic acid-Sephadex G-25 column at 0°-4°C. The void volume material was collected and its acidity adjusted to pH 4.7 with powdered sodium bicarbonate. The lyophilized SPDP derivatives of HCG and HRP were added to the gel filtered thiolated Ficoll$_{70}$ dilute solution. This reaction was carried out at room temperature utilizing an optimum molar ratio of hCG:Ficoll$_{70}$:HRP of 2:1:(4-15). At the end of this coupling reaction, a sufficient amount of S(2-pyridyl thiol) cysteine was added to the reaction mixture to transform remaining thiol groups on the Ficoll$_{70}$ to non-cross-linkable side chains. The resulting product was purified by gel filtration through a Sephacryl S-200 column in 0.2 molar ammonium sulfate-0.05 molar sodium phosphate solution, pH 6.8.

## EXAMPLE 4

A conjugate of hCG, hRP, and Ficoll$_{70}$ was prepared in the manner of Example 3 and the conjugate associated to $\gamma$-globulin sensitized nylon plates prepared in the manner of Example 1. To five rectangular test vessels were added 0.5 ml of urine from a non-pregnant woman. To the urine that was added to vessels Nos. 2, 3, 4, and 5, also was added 12.5, 25, 50, and 100 mIU of hCG (second International Reference Standard), respectively, per one milliliter of urine.

Conjugate-sensitized nylon plates were inserted into each vessel at the same time and allowed to stand at room temperature for 30 minutes. Each plate then was removed and discarded. Next, 0.5 ml of the substrate solution was added to each vessel. The substrate solution consisted of 0.05% amino antipyrine in a phenol solution (1.6% phenol in water) mixed at a 1:1 weight ratio with a 0.003% solution of $H_2O_2$ in 0.2 M $K_2HPO_4$ buffer, pH 7.0.

After ten minutes, vessel #1 showed no detectable color change of the test urine. Vessels #2 and #3 showed a deep pink color and vessels #4 and #5 showed a dark reddish-purple color.

These results demonstrate that the assay was able to detect as little as 12.5 mIU/ml of hCG in the urine and that no hCG was detected in the urine of the non-pregnant woman.

0155224

CLAIMS

1. A reagent for determination of one component of an antibody-antigen reaction or a receptor-receptor binding molecule reaction characterized by the reaction product selected from the group consisting of:

(a) a solid support matrix having the antibody linked thereto and a conjugate of the antigen, a carrier molecule, and a chromagenically-responsive marker;

(b) a solid support matrix having the antigen linked thereto and a conjugate of the antibody, a carrier molecule, and a chromagenically-responsive marker;

(c) a solid support matrix having the receptor linked thereto and a conjugate of the receptor binding molecule, a carrier molecule, and a chromagenically-responsive marker; and

(d) a solid support matrix having the receptor binding molecule linked thereto and a conjugate of the receptor, a carrier molecule, and a chromagenically-responsive marker.

2. The reagent of claim 1 comprising reaction product (a) or (b).

3. The reagent of claim 1 wherein mixtures of said components are used to form said reaction product.

4. The reagent of claim 1 wherein said matrix is selected from the group consisting of nylon, polybutadiene, polystyrene, butadiene-styrene copolymers, glass beads, agarose, cellulose, phospholipid bilayer membrane liposome, latex, dextran, and filter paper.

5. The reagent of claim 4 wherein said matrix is a nylon.

6. The reagent of claim 2 wherein said antigen is a gonadotropic hormone.

7. The reagent of claim 1 wherein said marker is selected from the group of an enzyme, a phenol, a heme, an enzyme cofactor, a dye, a chemiluminescence precursor, an enzyme substrate, a fluorochrome, a fluorogen, a fluorescent quencher, and mixtures thereof.

8. The reagent of claim 7 wherein said marker comprises an enzyme.

9. The reagent of claim 8 wherein said enzyme is peroxidase.

10. The reagent of claim 1 wherein the carrier molecule of said conjugate is a sucrose polymer, bovine serum albumin, or mixtures thereof.

11. The reagent of claim 10 wherein said carrier molecule of said conjugate comprises a sucrose polymer.

12. The reagent of claim 2 wherein said conjugate contains between about 1 and 15 molecules of marker per molecule of antigen or antibody.

13. The reagent of claim 2 wherein said conjugate contains between about 4 and 15 molecules of enzyme per molecule of antigen or antibody.

14. The reagent of claim 1 wherein said reaction product (a) comprises a nylon-6 support matrix having anti-$\beta$-human chorionic gonadotropin linked thereto which has been reacted with a conjugate comprising a sucrose polymer carrier molecule containing one or two molecules of human chorionic gonadotropin per molecule of sucrose polymer and between about 4 and 15 molecules of peroxidase per molecule of sucrose polymer.

15. A method for preparing a reagent which is useful in the determination of one component of an antibody-antigen reaction characterized by:
    (a) covalently linking an antibody to a solid support matrix;
    (b) separately forming a conjugate with a carrier molecule of said antigen and a chromagenically-responsive marker; and
    (c) reacting said antibody-modified solid support matrix with said conjugate.

16. The method of claim 15 wherein a mixture of said components is used to prepare said reagent.

17. The method of claim 15 wherein said solid support matrix comprises a nylon.

18. The method of claim 15 wherein said antigen and said antibody are interchanged and the reaction steps conducted.

19. The method of claim 15 wherein said antigen is a gonadotropin hormone.

20. The method of claim 15 wherein said chromagenically-responsive marker is selected from an enzyme, a phenol, a heme, and mixtures thereof.

21. The method of claim 20 wherein said marker is an enzyme.

22. The method of claim 21 wherein said enzyme is peroxidase.

23. The method of claim 15 wherein said conjugate contains one or two molecules of antigen and between about 4 and 15 molecules of marker per carrier molecule.

24. The method of claim 23 wherein said solid support matrix is a nylon, said carrier molecule is bovine serum albumin or a sucrose polymer, said marker is an enzyme, and said antigen is human chorionic gonadotropin.

25. A method for the immunochemical determination of a component of an antigen-antibody reaction characterized by:
(a) providing a reagent characterized by the reaction product of a solid support matrix having the antibody linked thereto and a conjugate with a carrier molecule of the antigen and a chromagenically-responsive marker;
(b) contacting a sample containing the component to be determined with said reagent for a time adequate for the immunochemical reaction to occur;
(c) removing said solid support matrix from contact with said sample;
(d) adding a chromagen responsive to said marker to said sample; and
(e) observing the sample to determine whether a color has appeared which color appearance is indicative of a threshold amount of said component to be determined in said sample.

26. The method of claim 23 wherein said solid support matrix is a nylon, said marker is an enzyme, and said antigen is a gonadotropin hormone.

27.    The method of claim 24 wherein said hormone is human chorionic gonadotropin.

28.    The method of claim 25 wherein said enzyme is a peroxidase.

29.    The method of claim 25 wherein said carrier molecule of said conjugate is a sucrose polymer.

30.    The method of claim 25 wherein a mixture of said components is used to prepare said reagent.

31.    The method of claim 30 wherein a mixture of chromagen is used in step (d).

32.    The method of claim 30 wherein step (d) is repeated sequentially with a different chromagen each repeat.

33.    The method of claim 25 for the determination of both estrogen and luteinizing hormone (LH) wherein said reagent (a) is formed by a mixture of estrogen and LH, their corresponding antibodies, and chromagenically-responsive markers; and a multiplicity of chromagens are added in step (d) as a mixture or sequentially.

34.    The method of claim 33 wherein said marker comprises alkaline phosphatase, and step (d) is conducted by sequentially adding p-nitrophenol and endoxylphosphate, in either order.

35.    In a reagent for the determination of a component of an antibody-antigen reaction or a receptor-ligand reaction of the type consisting of one component in an insolubilized form and the other component linked to an enzyme, the improvement wherein said reagent comprises:
        (a) said one component in an insolubilized form;
        (b) said other component provided in a conjugate of a carrier molecule bearing said second component and between about 4 and 15 molecules of a chromagenically-rsponsive marker molecule per molecule of said second component.

36.    The improved reagent of claim 35 wherein said chromagenically-responsive marker is an enzyme.

37. The improved reagent of claim 35 wherein said first component is an antibody or receptor and said second component is an antigen or ligand, respectively.

0155224

1/1

FIG. 1

MATRIX

CARRIER

hCG

hCG

hCG

HRP

FIG. 2

MATRIX

hCG

CARRIER

hCG

hCG

HRP

LEGEND:

MATRIX, eg. NYLON-6

ANTIBODY, eg. $\gamma$-GLOBULIN

REAGENT ANTIGEN, eg. hCG

CARRIER MOLECULE, eg. FICOLL$_{70}$

MARKER, eg. AN ENZYME LIKE HRP

ANTIGEN TO BE DETERMINED, eg. hCG FROM URINE OF PREGNANT WOMAN